# EUROPÄISCHE PATENTANMELDUNG

(11) **EP 0 803 497 A1**
(43) Veröffentlichungstag der Anmeldung: **29.10.1997**
(21) Anmeldenummer: 97103088.7
(22) Anmeldetag: 26.02.1997
(51) Int. Cl.: C07C 217/34, C07C 217/28, C08G 59/64

(54) **Polyamine, ein Verfahren zu ihrer Herstellung und ihre Verwendung als Härter in Epoxidharzen**

(30) Priorität: 23.04.1996 DE 19616020
(71) Anmelder: HÜLS AKTIENGESELLSCHAFT, 45764 Marl (DE)
(72) Erfinder: Wolf, Elmar, Dr., 45661 Recklinghausen (DE)

(57) **Zusammenfassung**

Polyamine der Formel I: 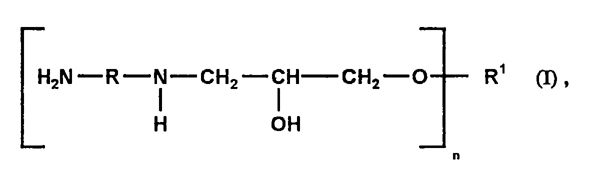
mit R: verzweigter (Cyclo)alkylen-Rest mit 2-22 Kohlenstoffatomen, wobei dieser auch 1 - 4 C₁₋₃-Alkyl-Gruppen und/oder 1 - 3 sek. Aminogruppen oder ein Sauerstoffatom aufweisen kann,
R¹: Alkyl-, Cycloalkyl-, Aryl-, Alkylaryl-Rest mit 4 - 24 Kohlenstoffatomen oder mit 1 - 4 CH₃-Gruppen verzweigter Alkylen- bzw. Arylen-Rest mit 4 - 24 Kohlenstoffatomen, wobei eine bis mehrere CH₂-Gruppen durch Sauerstoff ersetzt sein können;
n: 1, 2, 3.

## Beschreibung

Die vorliegende Erfindung betrifft neue Polyamine sowie ein Verfahren zu ihrer Herstellung.

Polyamine - in der Regel handelt es sich um (cyclo)aliphatische Diamine - stellen wertvolle Ausgangsprodukte zur Vernetzung von Epoxid-Harzen (EP-Harzen) dar. Sie weisen jedoch den Nachteil auf, daß sie einen merklichen Dampfdruck besitzen, so daß bei ihrem Verarbeiten die Umgebung durch besondere Maßnahmen geschützt werden muß.

Zur Einhaltung von immer niedrigeren MAK-Werten sowie zunehmend verschärften Umweltschutzauflagen müssen mittel- und langfristig alternative Polyamine entwickelt werden, die den bestehenden und weiter steigenden Ansprüchen genügen. Die Polyamine der vorliegenden Erfindung tragen diesen gesteigerten Ansprüchen Rechnung. Obendrein sind sie den Diaminen des Standes der Technik überlegen, da man durch sie in die Lage versetzt wird, in größerem Umfang als es bis jetzt möglich war, Kunststoffe "maßzuschneidern".

Gegenstand der vorliegenden Erfindung sind Polyamine der Formel I:
mit R: verzweigter (Cyclo)alkylen-Rest mit 2-22 Kohlenstoffatomen, wobei dieser auch 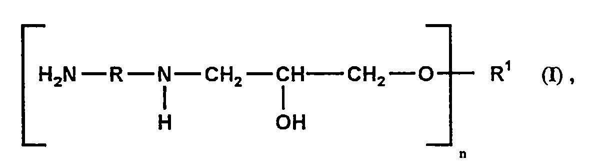 1 - 4 C₁₋₃-Alkyl-Gruppen und/oder 1 - 3 sek. Aminogruppen oder ein Sauerstoffatom aufweisen kann,
R¹: Alkyl-, Cycloalkyl-, Aryl-, Alkylaryl-Rest mit 4 - 24 Kohlenstoffatomen oder mit 1 - 4 CH₃-Gruppen verzweigter Alkylen- bzw. Arylen-Rest mit 4 - 24 Kohlenstoffatomen, wobei eine bis mehrere CH₂-Gruppen durch Sauerstoff ersetzt sein können;
n: 1, 2, 3.

Die erfindungsgemäßen Verbindungen zeichnen sich durch einen basischen Amingehalt von 3 - 12 mmol NH₂/g und ein NH-aktiv-Äquivalentgewicht von 60 - 150 aus. Ihre Viskosität variiert in einem weiten Bereich. Die erfindungsgemäßen Verbindungen können fest, hochviskos bis niedrigviskos sein. Neben der sicheren Handhabung weisen die erfindungsgemäßen Verbindungen noch eine Reihe weiterer Vorteile auf wie z. B. verringerte Carbamatbildung sowie verbesserter Verlauf der damit bei Raumtemperatur ausgehärteten Epoxidgemische.

Die erfindungsgemäßen Polyamine (I) sind erhältlich durch Umsetzung einer Epoxidverbindung (III) mit einem mindestens zwei NH₂-Gruppen aufweisenden Polyamin (II).

Die erfindungsgemäß einsetzbaren Polyamine (II) stammen aus der Gruppe der aliphatischen, cyclischen oder (cyclo)aliphatischen primären und gegebenenfalls sekundären Aminogruppen enthaltende Polyaminen, wie z. B. Ethylendiamin, Diethylentriamin, Triethylentetramin, Hexamethylendiamin, 2-Methylpentamethylendiamin, 2.2.4(2.4.4)-Trimethylhexamethylendiamin, Bis-(4-amino-3-methyl-cyclohexyl)-methan, Bis-(p-aminocyclohexyl)-methan, Isophorondiamin (IPD), 1.2-Diaminocyclohexan, 1.3-Bis-(aminomethyl)-cyclohexan, m-Xylylendiamin.

Die erfindungsgemäß geeigneten Epoxidverbindungen (III) sind 1 - 3 Epoxidgruppen enthaltende Verbindungen, wie z. B. n-Butyl-, 2-Ethylhexyl-, Octyl-, Dodecyl-, Phenyl-, p-tert.-Butylphenylglycidether, Hexandiol-, Butandiol-, 1.4-Cyclohexyldimethanol-, Neopentylglykol-, Diethylenglykoldiglycidether, Diglycidylether des Bisphenol A Glycerintriglycidether und Trimethylolpropantriglycidether.

Beim erfindungsgemäßen Verfahren wird die EP-Verbindung (III) dem Polyamin (II) bei 50 - 120 °C, vorzugsweise 80 - 100 °C, im Molverhältnis 1:5-30 unter intensivem Rühren zugegeben. Das nicht umgesetzte Polyamin (II) wird bei 80 - 140 °C unter reduziertem Druck vom Reaktionsprodukt (I) durch eine Dünnschichtdestillation abgetrennt.

Die Aushärtung von flüssigen Epoxidharzen mit den erfindungsgemäßen Polyaminen ist auch bei niederen Temperaturen von z. B. 5 °C und bei hoher atmosphärischer Feuchtigkeit möglich wobei die Filme keine Ausschwitzerscheinungen oder andere Störungen an der Oberfläche aufweisen. Die Flexibilität und Haftfestigkeit der ausgehärteten Filme aufweisen und Beton ist gut.

Die härtbaren Systeme aus den erfindungsgemäßen Polyaminen und flüssigen Epoxidharzen können weitere übliche Zusätze, wie Füllstoffe, Pigmente, Weichmacher, enthalten. Außer als Lacke und Beschichtungsmittel können sie auch als Klebstoffe, Imprägnier-, Tauch- und Gießharze, speziell in der Elektrotechnik, dienen.

### Experimenteller Teil

### Beispiel 1

a) Zu 3400 Gew.-T. Isophorondiamin wurden bei 80 °C 380 Gew.-T. Bisphenol A-diglycidether so zugegeben, daß die Temperatur nicht über 100 °C stieg. Nach Beendigung der EP-Zugabe wurde zur Vervollständigung der Reaktion noch ca. 2 h bei 100 °C weitererhitzt.
b) Das nicht umgesetzte IPD wurde im Dünnschichtverdampfer bei 100 °C/0,1 mbar vom Reaktionsprodukt abgetrennt. Das Reaktionsprodukt hatte folgende Kenndaten:

| | |
|---|---|
| IPD [%]: | 0,3 |
| NH₂ [mmol/g]: | 5,4 |
| NH-aktiv-Äquivalentgewicht: | 121 |
| Smp. [°C]: | 62 - 70 |

### Beispiel 2

a) 3160 Gew.-T. 2.2.4(2.4.4)-Trimethylhexamethylendiamin wurden mit 380 Gew.-T. Bisphenol A-diglycidether entsprechend dem in 1a beschriebenen Verfahren umgesetzt.
b) Die Abtrennung des nicht umgesetzten TMD erfolgte bei 95 °C/0,1 mbar. Das Reaktionsprodukt hatte folgende Kenndaten:

| | | |
|---|---|---|
| TMD [%]: | | 0,1 |
| NH₂ [mmol/g]: | | 5,5 |
| NH-aktiv-Äquivalentgewicht: | | 117 |
| Viskosität [mPa·s] | 23 °C: | 2,3·10⁶ |
| | 40 °C: | 1,45·10⁵ |
| | 60 °C: | 5,9·10⁴ |

### Beispiel 3

a) 3400 Gew.-T. IPD wurden mit 230 Gew.-T. Hexandioldiglycidether analog zum Beispiel 1a umgesetzt.
b) Das nicht umgesetzte IPD wird bei 100 °C/0,1 mbar vom Reaktionsprodukt abgetrennt. Das Reaktionsprodukt hatte folgende Kenndaten:

| | |
|---|---|
| IPD [%]: | < 0,1 |
| NH₂ [mmol/g]: | 7,1 |
| NH-aktiv-Äquivalentgewicht: | 96 |
| Viskosität (23 °C) [mPa·s]: | 35·10³ |

### Beispiel 4

a) 2320 Gew.-T. 2-Methylpentamethylendiamin wurden mit 230 Gew.-T. Hexandioldiglycidether analog zum Beispiel 1a umgesetzt.
b) Das nicht umgesetzte 2-Methylpentamethylendiamin wird bei 80 °C/0,1 mbar vom Reaktionsprodukt abgetrennt. Das Reaktionsprodukt hatte folgende Kenndaten:

| | |
|---|---|
| 2-Methylpentamethylendiamin [%]: | < 0,1 |
| NH₂ [mmol/g]: | 8,5 |
| NH-aktiv-Äquivalentgewicht: | 78 |
| Viskosität (23 °C) [mPa·s]: | 15·10³ |

### Beispiel 5

a) 1160 Gew.-T. 2-Methylpentamethylendiamin wurden mit 186 Gew.-T. 2-Ethylhexylglycidether analog zum Beispiel 1a umgesetzt.
b) Das nicht umgesetzte 2-Methylpentamethylendiamin wird bei 80 °C/0,1 mbar vom Reaktionsprodukt abgetrennt. Das Reaktionsprodukt hatte folgende Kenndaten:

| | |
|---|---|
| 2-Methylpentamethylendiamin [%]: | 0,1 |
| NH₂ [mmol/g]: | 6,4 |
| NH-aktiv-Äquivalentgewicht: | 103 |
| Viskosität (23 °C) [mPa·s]: | 560 |

## Patentansprüche

1. Polyamine der Formel I: 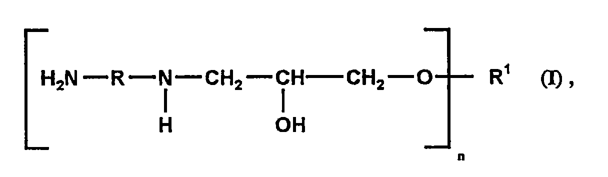
mit R: verzweigter (Cyclo)alkylen-Rest mit 2-22 Kohlenstoffatomen, wobei dieser auch 1 - 4 C₁₋₃-Alkyl-Gruppen und/oder 1 - 3 sek. Aminogruppen oder ein Sauerstoffatom aufweisen kann,
R¹: Alkyl-, Cycloalkyl-, Aryl-, Alkylaryl-Rest mit 4 - 24 Kohlenstoffatomen oder mit 1 - 4 CH₃-Gruppen verzweigter Alkylen- bzw. Arylen-Rest mit 4 - 24 Kohenstoffatomen, wobei eine bis mehrere CH₂-Gruppen durch Sauerstoff ersetzt sein können;
n: 1, 2, 3.

2. Polyamine nach Anspruch 1,
dadurch gekennzeichnet,
daß R Kohlenwasserstoff-Reste der folgenden Verbindungen bedeutet: Ethylendiamin, Diethylentriamin, Triethylentetramin, Hexamethylendiamin, 2-Methyl-pentamethylendiamin, 2.2.4(2.4.4)-Trimethylhexamethylendiamin, Bis-(4-amino-3-methyl-cyclohexyl)-methan, Bis-(p-aminocyclohexyl)-methan, Isophorondiamin (IPD), 1.2-Diaminocyclohexan, 1.3-Bis-(aminomethyl)-cyclohexan, m-Xylylendiamin.

3. Polyamine nach den Ansprüchen 1 bis 2,
dadurch gekennzeichnet,
daß R¹ Reste von n-Butyl-, 2-Ethylhexyl-, Octyl-, Dodecyl-, Phenyl-, p-tert.-Butylphenylglycidether, Hexandiol-, Butandiol-, 1.4-Cyclohexyldimethanol-, Neopentylglykol-, Diethylenglykoldiglycidether, Diglycidylether des Bisphenol A, Glycerintriglycidether und Trimethylolpropantriglycidether bedeutet.

4. Verfahren zur Herstellung der erfindungsgemäßen Verbindungen gemäß den Ansprüchen 1 bis 3,
dadurch gekennzeichnet,
daß Polyamine (II) mit Epoxidverbindungen (III) im Molverhältnis 5 - 30 : 1 bei 50 bis 120 °C umgesetzt werden: 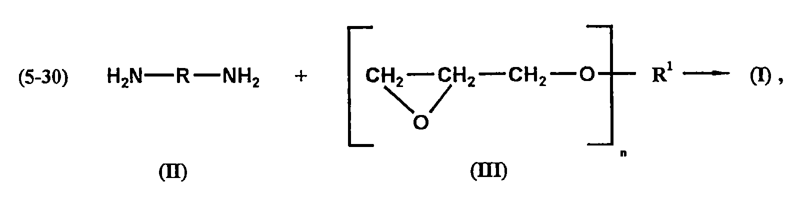 wobei R, R¹, n die im Anspruch 1 angegebene Bedeutung haben, und nach erfolgter Umsetzung das nicht umgesetzte Polyamin (II) bei 80 - 140 °C und reduziertem Druck durch eine Dünnschichtdestillation vom Reaktionsprodukt abgetrennt wird.

5. Verfahren nach Anspruch 4,
dadurch gekennzeichnet,
daß als Polyamine II Ethylendiamin, Diethylentriamin, Triethylentetramin, Hexamethylendiamin, 2-Methyl-pentamethylendiamin, 2.2.4(2.4.4)-Trimethylhexamethylendiamin, Bis-(4-amino-3-methyl-cyclohexyl)-methan, Bis-(p-aminocyclohexyl)-methan, Isophorondiamin (IPD), 1.2-Diaminocyclohexan, 1.3-Bis-(aminomethyl)-cyclohexan, m-Xylylendiamin eingesetzt werden.

6. Verfahren nach den Ansprüchen 4 bis 5,
dadurch gekennzeichnet,
daß als Epoxidverbindungen n-Butyl-, 2-Ethylhexyl-, Octyl-, Dodecyl-, Phenyl-, p-tert.-Butylphenylglycidether, Hexandiol-, Butandiol-, 1.4-Cyclohexyldimethanol-, Neopentylglykol-, Diethylenglykoldiglycidether, Diglycidylether des Bisphenol A, Glycerintriglycidether und Trimethylolpropantriglycidether eingesetzt werden.

7. Verwendung der erfindungsgemäßen Polyamine (I) nach den Ansprüchen 1 bis 3 in Lacken, Beschichtungsmitteln, Klebstoffen, Imprägnier-, Tauch- und Gießharzen.
